(19) Europäisches Patentamt
European Patent Office
Office européen des brevets

(11) Veröffentlichungsnummer: **0 052 200**
**B1**

(12) # EUROPÄISCHE PATENTSCHRIFT

(45) Veröffentlichungstag der Patentschrift: **06.06.84**

(51) Int. Cl.[3]: **C 07 D 317/30**, C 07 D 319/06, C 07 D 209/20

(21) Anmeldenummer: **81107463.2**

(22) Anmeldetag: **19.09.81**

(54) **Cyclische Acetale von N-Acylglutaminsäure-gamma-semialdehyden, Verfahren zu deren Herstellung und ihre Verwendung.**

(30) Priorität: **15.11.80 DE 3043252**

(43) Veröffentlichungstag der Anmeldung: **26.05.82 Patentblatt 82/21**

(45) Bekanntmachung des Hinweises auf die Patenterteilung: **06.06.84 Patentblatt 84/23**

(84) Benannte Vertragsstaaten: **BE CH DE FR GB LI NL**

(56) Entgegenhaltungen:
**GB - A - 2 001 309**
**US - A - 2 557 920**

**Chemical Abstracts, Band 91, Nr. 3, 16. Juli 1979, Columbus, Ohio, USA, Seite 716, Spalte 1, Abstract Nr. 21113h**
**Patent Abstracts of Japan Band 4, Nr. 106, 30. Juli 1980, Seite 157C20**
**Agric. Biol. Chem. 42(4), 843-845, 1978**

(73) Patentinhaber: **Degussa Aktiengesellschaft, Weissfrauenstrasse 9, D-6000 Frankfurt am Main 1 (DE)**

(72) Erfinder: **Kleemann, Axel, Dr. Dipl-Chem., Greifenhagenstrasse 25, D-6450 Hanau 9 (DE)**
Erfinder: **Samson, Marc, Dr., Grünaustrasse 1, D-6450 Hanau 9 (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Gegenstand der Erfindung sind cyclische Acetale von N-Acylglutaminsäure-γ-semialdehyden der Formel

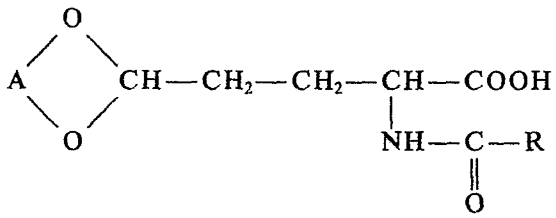

(I)

in der A eine unsubstituierte oder durch 1 bis 2 Methylgruppen substituierte Alkylengruppe mit 2 oder 3 Kohlenstoffatomen und R einen Methyl-, Methoxy-, Phenyl- oder Benzyloxyrest bedeuten, und ein Verfahren zu ihrer Herstellung.

Diese cyclischen Acetale von N-Acylglutaminsäure-γ-semialdehyden sind wertvolle Zwischenprodukte für die Herstellung der entsprechenden N-Acyltryptophane. Ein weiterer Gegenstand der Erfindung ist daher ihre Verwendung zur Herstellung der entsprechenden N-Acyltrytophane.

L-Trytophan ist eine essentielle Aminosäure, die häufig in Futtermitteln und Mischfuttern die limitierende Aminosäure darstellt. Da L-Trytophan durch enzymatische Spaltung von D,L-N-Acyltrytophanen gewonnen werden kann, kommt deren Synthese eine große Bedeutung zu.

Aus Agric. Biol. Chem., 42 (4), 843–845, 1978 ist es zwar bereits bekannt, daß N-Acetyl-L-tryptophan durch Umsetzung von enzymatisch gewonnenem N-Acetyl-L-glutaminsäure-γ-semialdehyd mit Phenylhydrazinhydrochlorid hergestellt werden kann. Die dabei erhaltene Ausbeute an N-Acetyl-L-tryptophan ist jedoch wenig befriedigend.

Cyclische Acetale von N-Acylglutaminsäure-γ-semialdehyden der allgemeinen Formel (I) können hergestellt werden nach einem Verfahren, welches dadurch gekennzeichnet ist, daß man

a) eine Verbindung der allgemeinen Formel

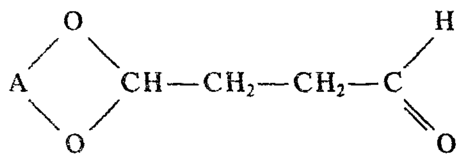

(II)

in der A wieder die bereits angegebene Bedeutung hat, in an sich bekannter Weise in wäßriger oder wäßrig-alkoholischer Lösung mit Cyanwasserstoff oder einer Cyanidionen liefernden Verbindung, Ammoniak oder einer Ammoniumionen liefernden Verbindungen und Kohlendioxid oder einer Carbonationen liefernden Verbindung zur Reaktion bringt,

b) das in Stufe a) erhaltene Reaktionsgemisch unter basischen Reaktionsbedingungen hydrolysiert und

c) das in Stufe b) erhaltene Hydrolysegemisch mit einer geeigneten acylierenden Verbindung umsetzt.

Die drei Reaktionsstufen a), b) und c) des erfindungsgemäßen Verfahrens verlaufen mit hohen Umsätzen.

Da auch die einzusetzenden Verbindungen der allgemeinen Formel (II) durch Hydroformylierung der entsprechenden 2-Vinyl-1,3-dioxolane bzw. 2-Vinyl-1,3-dioxane und letztere durch Acetalisierung von Acrolein mit den entsprechenden 1,2- bzw. 1,3-Glykolen leicht und in hohen Ausbeuten erhältlich sind, können die cyclischen Acetale von N-Acylglutaminsäure-γ-semialdehyden der allgemeinen Formel (I) kostengünstig hergestellt werden. Da die Verbindungen der allgemeinen Formel (I) dann ebenfalls leicht mit hohen Ausbeuten in die entsprechenden N-Acyltryptophane der allgemeinen Formel

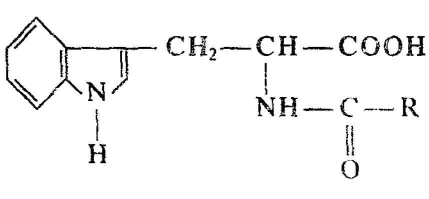

(III)

umgewandelt werden können, in der R wieder die bereits angegebene Bedeutung hat, wird insgesamt

ein neuer, von Acrolein ausgehender, vorteilhafter und kostengünstiger Weg zu diesen N-Acyltryptophanen und damit zum L-Tryptophan eröffnet.

Beispiele für einzusetzende Verbindungen der allgemeinen Formel (II) sind 2-(2'-Formyläthyl)-1,3-dioxolan, 2-(2'-Formyläthyl)-1,3-dioxan oder 2-(2'-Formyläthyl)-5,5-dimethyl-1,3-dioxan.

Die Verbindungen der allgemeinen Formel (II) werden in einer ersten Reaktionsstufe in der für die Bildung von Hydantoinen aus Aldehyden an sich bekannter Weise mit Cyanwasserstoff oder einer Cyanidionen liefernden Verbindung, wie Natriumcyanid oder Kaliumcyanid, mit Ammoniak oder einer Ammoniumionen liefernden Verbindung, wie Ammoniumhydroxid oder Ammoniumchlorid, und mit Kohlendioxid oder einer Carbonationen liefernden Verbindung, wie Natrium- oder Kaliumbicarbonat, -carbonat oder -carbamat, umgesetzt. Es können auch Verbindungen eingesetzt werden, die gleichzeitig Cyanid- und Ammoniumionen liefern, wie Ammoniumcyanid, oder die gleichzeitig Ammonium- und Carbonationen liefern, wie Ammoniumcarbonat oder Ammoniumcarbamat.

Die Umsetzung in der ersten Reaktionsstufe erfolgt in Wasser oder in einem Gemisch aus Wasser und Methanol oder Äthanol. Sie kann in einem weiten Temperaturbereich vorgenommen werden. Bevorzugt wird eine Temperatur zwischen 30 und 90°C, weil in diesem Bereich eine zufriedenstellende Reaktionsgeschwindigkeit erreicht wird und der eventuell erforderliche Überdruck technisch kein Hindernis bildet.

Die Mengen der einzelnen Reaktionsteilnehmer können innerhalb weiter Grenzen variiert werden. Vorzugsweise werden je Mol Verbindung der allgemeinen Formel (II) 1 bis 1,5 Mol Cyanwasserstoff oder einer Cyanidionen liefernden Verbindung, 2 bis 15 Mol Ammoniak oder einer Ammoniumionen liefernden Verbindung und 1 bis 2 Mol Kohlendioxid oder einer Carbonationen liefernden Verbindung eingesetzt. Die Verbindungen der allgemeinen Formel (II) können gleichzeitig mit allen drei anderen Reaktionsteilnehmern umgesetzt werden. Ebenso ist es aber auch möglich, sie zunächst nur mit der Cyanidkomponente und anschließend mit den beiden anderen Komponenten gleichzeitig, oder zunächst nur mit der Cyanidkomponente, dann nur mit der Ammoniumkomponente und dann erst mit der Kohlendioxid- bzw. Carbonatkomponente umzusetzen. Besonders vorteilhaft ist es, wenn die Verbindung der allgemeinen Formel (II) in Methanol oder Äthanol gelöst und diese Lösung langsam zu einer auf die gewünschte Reaktionstemperatur erwärmten wäßrigen Lösung oder Suspension der anderen Reaktionsteilnehmer zudosiert wird. Zur Erzielung eines hohen Umsatzes ist eine angemessene Nachreaktionszeit von beispielsweise etwa 5 Stunden nach dem Ende der Dosierung zu empfehlen.

Je nach den angewandten Reaktionsbedingungen enthält das Reaktionsgemisch nach der Durchführung der ersten Reaktionsstufe neben dem zu erwartenden Hydantoin der allgemeinen Formel

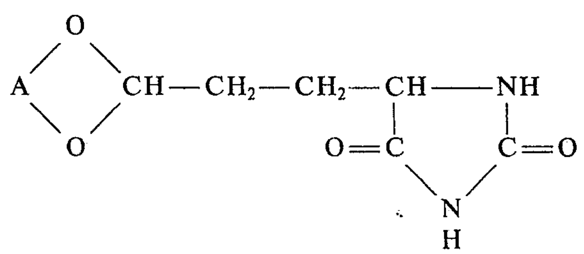
(IV)

auch noch einen mehr oder weniger großen Anteil an dem $\alpha$-N-Carbamoyl-carbonsäureamid der allgemeinen Formel

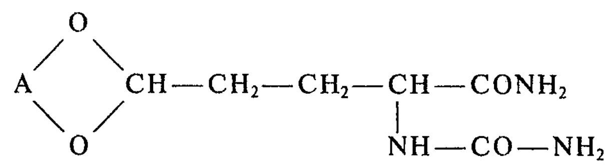
(V)

wobei in den Formeln (IV) und (V) A wieder die oben bereitsangegebene Bedeutung hat.

Eine Trennung der beiden Reaktionsprodukte ist jedoch nicht erforderlich, da sie beide bei der nachfolgenden Reaktionsstufe b) zu den cyclischen Acetalen des Glutaminsäure-$\gamma$-semialdehyds umgesetzt werden. Es kann aber zweckmäßig sein, vor Durchführung der zweiten Reaktionsstufe b) die im rohen Reaktionsgemisch der ersten Reaktionsstufe a) enthaltenen Ammoniumsalze zu verkochen, den gegebenenfalls enthaltenen Alkohol abzudestillieren und das Reaktionsgemisch unter vermindertem Druck einzuengen.

In der Reaktionsstufe b) wird nun das in Stufe a) erhaltene Gemisch von Verbindungen der allgemeinen Formeln (IV) und (V) in der für die Bildung von $\alpha$-Aminosäuren aus den entsprechenden substituierten Hydantoinen an sich bekannter Weise unter basischen Hydrolysebedingungen umgesetzt. Bevorzugt werden Alkali- oder Erdalkalimetallhydroxide oder Alkalimetallcarbonate in wäßrigem Medium verwendet. Beispielsweise können mit gutem Erfolg NaOH, $Na_2CO_3$, $K_2CO_3$, $Ca(OH)_2$ oder $Ba(OH)_2$ eingesetzt werden. Die Reaktionstemperatur kann in weiten Grenzen zwischen 20°C und 200°C variiert

werden. Bevorzugt werden Temperaturen zwischen 100°C und 150°C. da in diesem Bereich zufriedenstellende Reaktionsgeschwindigkeiten erreicht werden. Besonders bevorzugte Verseifungsbedingungen sind Temperaturen zwischen 130° und 150°C, Reaktionszeiten von 0,5 bis 1 Stunde und ein Molverhältnis von Substrat zu Base von 1 : 2,2.

Das Hydrolysegemisch nach der Durchführung der Verfahrensstufe b) enthält eine wäßrige Lösung des entsprechenden Salzes von Glutaminsäure-γ-semialdehydacetal. Dieses Hydrolysegemisch kann direkt für die Verfahrensstufe c) eingesetzt werden. Es kann aber zweckmäßig sein, vor Durchführung der dritten Reaktionsstufe den im rohen Hydrolysegemisch gelösten Ammoniak durch Erhitzen bei Normaldruck oder im Vakuum zu entfernen.

In der Reaktionsstufe c) wird die in Stufe b) erhaltene wäßrige Lösung der Glutaminsäure-γ-semialdehydacetale mit geeigneten acylierenden Reagenzien umgesetzt. Beispiele für die in wäßrigem Medium einzusetzenden acylierenden Reagenzien sind Acetylchlorid, Essigsäureanhydrid, Chlorameisensäuremethylester, Benzoylchlorid oder Benzyloxycarbonylchlorid.

Die Reaktionsstufe c) wird zweckmäßigerweise bei einem pH oberhalb 7 durchgeführt. Je nach Durchführung der Verfahrensstufe b) kann es erforderlich sein, den pH durch Zugabe von Base während der Reaktion nachzustellen. Die Reaktionstemperatur kann innerhalb weiter Grenzen variiert werden. Zweckmäßig sind Temperaturen zwischen 0 und 80°C, vorzugsweise zwischen 0 und 25°C. Die acylierenden Reagenzien können im Überschuß eingesetzt werden. Aus wirtschaftlichen Gründen ist es jedoch vorteilhafter, nur die der in der Reaktionsstufe a) eingesetzten Menge an der Verbindung der allgemeinen Formel (II) äquivalente Menge anzuwenden.

Die acylierenden Reagenzien können auf einmal zu dem aus der Reaktionsstufe b) erhaltenen Gemisch zugesetzt werden. Es ist aber vorteilhafter, die acylierenden Reagenzien langsam zu dem aus der Reaktionsstufe b) erhaltenen Gemisch zuzudosieren. In beiden Fällen ist eine Reaktionszeit von insgesamt etwa 1 Stunde im allgemeinen ausreichend.

Da die gebildeten cyclischen Acetale der N-Acylglutaminsäure-γ-semialdehyde meistens bei Raumtemperatur in Wasser schwerlöslich sind, können sie durch einfaches Abfiltrieren praktisch vollständig und in hoher Reinheit erhalten werden.

Um aus den N-Acylglutaminsäure-γ-semialdehydacetalen N-Acyltryptophane herzustellen, werden sie bei einem pH zwischen 0,1 und 4, vorzugsweise zwischen 1 und 3, mit Phenylhydrazin umgesetzt. Der erforderliche pH kann durch eine anorganische Säure, wie Schwefelsäure oder Phosphorsäure, durch eine organische Säure, wie Oxalsäure, Ameisensäure, Essigsäure, Benzolsulfonsäure oder p-Toluolsulfonsäure, oder durch einen stark sauren Ionenaustauscher eingestellt werden. Bevorzugt wird die Verwendung von Salzsäure.

Die Reaktionstemperatur kann innerhalb weiter Grenzen variiert werden. Zweckmäßig sind Temperaturen zwischen 60 und 150°C, vorzugsweise zwischen 70 und 120°C.

Das Phenylhydrazin kann im Überschuß eingesetzt werden. Aus wirtschaftlichen Gründen ist jedoch vorteilhafter, nur die dem eingesetzten N-Acylglutaminsäure-γ-semialdehydacetal äquivalente Menge anzuwenden.

Das Phenylhydrazin kann mit dem N-Acylglutaminsäure-γ-semialdehydacetal und der erforderlichen Menge an Säure vermengt und auf die gewünschte Reaktionstemperatur erhitzt werden. Ebenso ist es aber auch möglich, eine saure Lösung des Phenylhydrazins vorzulegen, zu erhitzen und eine Lösung des N-Acylglutaminsäure-γ-semialdehydacetals zuzudosieren. In beiden Fällen ist eine Reaktionszeit von insgesamt 3 bis 4 Stunden im allgemeinen ausreichend. Nach beendeter Reaktion kann die erhaltene Lösung des N-Acyltryptophans eingeengt und der Rückstand umkristallisiert werden. Man kann aber auch durch Abkühlen der Lösung das N-Acyltryptophan zur Kristallisation bringen und abtrennen.

Durch die nachfolgenden Beispiele soll die Erfindung näher verdeutlicht werden. Sofern nicht anders angegeben, bedeuten die Prozentangaben Gewichtsprozente.

## Beispiel 1

Zu einer Suspension aus 48 g Ammoniumcarbonat, 5,1 g Blausäure und 110 ml wäßrigem Ammoniak (25%ig) werden bei 35°C im Verlaufe einer Stunde 16,2 g 2-(2'-Formyläthyl)-1,3-dioxolan zugetropft und es wird 5 Stunden bei 40°C nachgerührt. Anschließend werden durch Temperaturerhöhung (bis 100°C Kopftemperatur) die Salze verkocht. Die verbleibende wäßrige Lösung wird mit 11,9 g Natriumhydroxid versetzt und 45 Stunden lang auf 140°C erhitzt. Nach dem Abkühlen wird die Reaktionslösung im Vakuum kurz eingeengt und innerhalb von 30 Minuten bei 0 bis 5°C mit 48,9 ml einer 50%igen Lösung von Chlorameisensäurebenzylester in Toluol versetzt. Nach 30 Minuten nachrühren wird die Lösung mit konzentrierter Salzsäure auf pH 2 eingestellt, wobei sich ein farbloses Öl abscheidet, das bei weiterem Stehenlassen auskristallisiert. Die Kristalle werden abfiltriert, mit Wasser nachgewaschen und im Vakuum getrocknet. Durch Umkristallisation aus Benzol erhält man 24,2 g N-Benzyloxycarbonylglutaminsäure-γ-semialdehydäthylenacetal.

Elementaranalyse: $C_{15}H_{19}NO_6$

| | | | |
|---|---|---|---|
| Berechnet: | C 58,24%, | H 6,19%, | · N 4,53%, |
| Gefunden: | C 58,80%, | H 6,11%, | N 4,56%. |

$^1$H-NMR-Spektrum ($CDCl_3$):

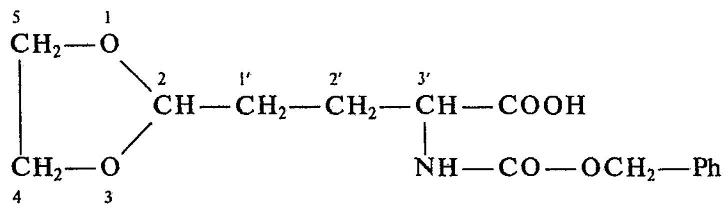

δ = 1,6–2,1 (m,4 H): H-1', H-2'
δ = 3,85 (m, 4 H): H-4, H-5
δ = 4,4 (m, 1 H): H-3'
δ = (t, 1 H): H-2
δ = 5,1 (s, 2 H): $-OCH_2-$
δ = 5,6 (d, 1 H): NH
δ = 7,35 (s, 5 H): Aromatische H's
δ = 10,3 (s, 1 H): COOH

Beispiel 2

Zu einer Suspension aus 45 g Ammoniumcarbonat, 5,1 g Blausäure und 100 ml wäßrigem Ammoniak (25%ig) werden bei 40°C im Verlaufe einer Stunde 16,3 g 2-(2'-Formyläthyl)-1,3-dioxolan zugetropft und es wird fünf Stunden bei dieser Temperatur nachgerührt. Anschließend werden durch Temperaturerhöhung (bis 100°C Kopftemperatur) die Salze verkocht. Die verbleibende wäßrige Lösung wird mit 15,4 g Kaliumhydroxid versetzt und 50 Minuten lang auf 150°C erhitzt. Nach dem Abkühlen wird die Reaktionsmischung im Vakuum kurz eingeengt und innerhalb von 40 Minuten bei 5°C mit 18 g Benzoylchlorid versetzt. Nach 30 Minuten Nachrühren wird die Lösung mit konzentrierter Salzsäure auf pH 2 eingestellt, wobei sich Kristalle abscheiden. Der Niederschlag wird abfiltriert, mit Wasser nachgewaschen und im Vakuum getrocknet. Durch Umkristallisation aus Benzol erhält man 31,8 g N-Benzoylglutaminsäure-γ-semialdehydäthylenacetal (Schmelzpunkt 136–137°C).

Elementaranlalyse: $C_{14}H_{17}NO_5$

| | | | |
|---|---|---|---|
| Berechnet: | C 60,21%, | H 6,13%, | N 5,02%, |
| Gefunden: | C 59,72%, | H 6,03%, | N 4,98%. |

$^1$H-NMR-Spektrum (DMSO-$d_6$/$CDCl_3$)

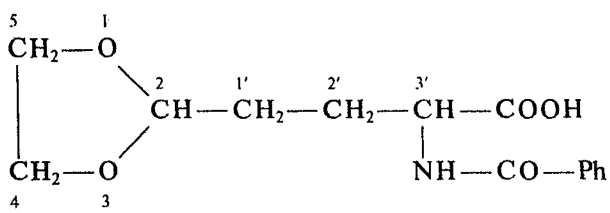

δ = 1,7–2,3 (m, 4 H): H-1', H-2'
δ = 3,9 (m, 4 H): H-4, H-5
δ = 4,65 (m, 1 H): H-3'
δ = 4,85 (t, 1 H): H-2
δ = 7,4; 7,9 (m, 5 H): Aromatische H's
δ = 7,9 (d, 1 H): NH

Beispiel 3

Zu einer Suspension aus 18 g Ammoniumcarbonat, 2,0 g Blausäure und 40 ml wäßrigem Ammoniak (25%ig) werden bei 40°C im Verlaufe einer halben Stunde 6,5 g 2-(2'-Formyläthyl)-1,3-dioxolan zugetropft und es wird drei Stunden bei 55°C nachgerührt. Anschließend werden durch Temperaturerhöhung (bis 100°C Kopftemperatur) die Salze verkocht. Die verbleibende wäßrige Lösung wird mit 4,7 g

Natriumhydroxid versetzt und 45 Minuten auf 140°C erhitzt. Nach dem Abkühlen wird die Reaktionslösung im Vakuum kurz eingeengt und innerhalb von 30 Minuten bei 5°C mit 7 ml Essigsäureanhydrid versetzt. Nach 30 Minuten Nachrühren wird die Lösung mit konzentrierter Salzsäure auf pH 2 eingestellt. Die Reaktionsmischung wird im Vakuum eingeengt und mit 150 ml Äthanol versetzt. Die unlöslichen Salze werden abfiltriert und das Filtrat eingeengt und im Vakuum getrocknet. Man erhält 9,5 g N-Acylglutaminsäure-γ-semialdehydäthylenacetal.

Elementaranalyse: $C_9H_{15}NO_5$

| | | | |
|---|---|---|---|
| Berechnet: | C 49,8%, | H 6,9%, | N 6,5%, |
| Gefunden: | C 49,7%, | H 6,8%, | N 6,6%. |

$^1$H-NMR-Spektrum (DMSO-$d_6$/$CDCl_3$)

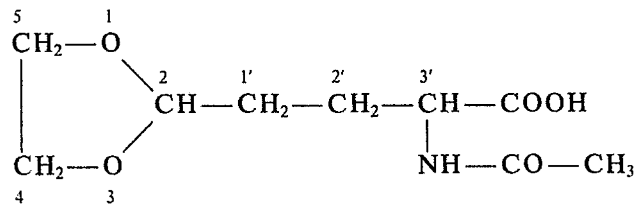

δ = 1,5–2,1 (m, 4 H): H-1', H-2'.
δ = 1,95 (s, 3 H): Acetyl
δ = 3,8 (m, 4 H): H-4, H-5
δ = 4,2 (m, 1 H): H-3'
δ = 4,8 (t, 1 H): H-2
δ = 7,6 (d, 1 H): N<u>H</u>
δ = 12,2 (bs, 1 H): COO<u>H</u>

Beispiel 4

Zu einer Suspension aus 42 g Ammoniumcarbonat, 6 g Blausäure und 120 ml wäßrigem Ammoniak (25%ig) wird bei 50°C im Verlaufe einer Stunde eine Lösung von 20,7 g 2-(2'-Formyläthyl)-1,3-dioxan in 50 ml Methanol zugetropft und es wird noch drei Stunden lang bei 50°C nachgerührt. Anschließend werden bis zu einer Kopftemperatur von 100°C das Methanol abdestilliert und die Salze verkocht. Die verbleibende wäßrige Lösung wird mit 11,5 g Natriumhydroxid versetzt und 40 Minuten auf 160°C erhitzt. Nach dem Abkühlen wird die Reaktionsmischung im Vakuum kurz eingeengt und bei 5°C mit 20 ml Essigsäureanhydrid versetzt. Nach 30 Minuten Nachreaktion wird die Lösung mit konzentrierter Salzsäure auf pH 2 eingestellt und mit 2 × 120 ml Chloroform extrahiert. Die organische Phase wird über Natriumsulfat getrocknet, abfiltriert und eingeengt. Der erhaltene Rückstand wird aus Benzol umkristallisiert. Ausbeute: 24,3 g (Schmelzpunkt: 133–134°C).

Elementaranalyse: $C_{10}H_{17}NO_5$

| | | | |
|---|---|---|---|
| Berechnet: | C 51,94%, | H 7,41%, | N 6,06%, |
| Gefunden: | C 52,45%, | H 7,28%, | N 6,03%. |

$^1$H-NMR-Spektrum (DMSO-$d_6$/$CDCl_3$)

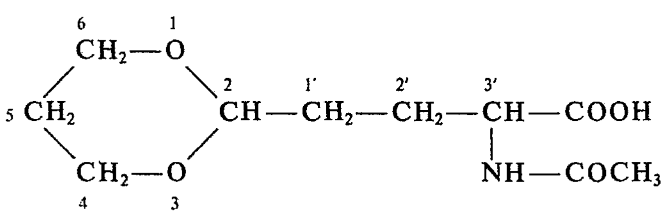

δ = 1,20–2,0 (m, 6 H): H-1', H-2', H-5
δ = 1,85 (s, 3 H): Acetyl
δ = 3,5–4,2 (m, 5 H): H-4, H-6, H-3'
δ = 4,45 (t, 1 H): H-2
δ = 8,0 (d, 1 H): N<u>H</u>
δ = 11,4 (bs, 1 H): COO<u>H</u>

## Beispiel 5

Zu einer Suspension aus 21 g Ammoniumcarbonat, 3 g Blausäure und 70 ml wäßrigem Ammoniak (25%ig) wird bei 50°C im Verlaufe einer Stunde eine Lösung von 10,4 g 2-(2'-Formyläthyl)-1,3-dioxan in 40 ml Methanol zugetropft und es wird noch drei Stunden bei 50°C nachgerührt. Anschließend werden das Methanol abdestilliert und die Salze verkocht. Die verbleibende wäßrige Lösung wird mit 9,3 g Kaliumhydroxid versetzt und 45 Minuten auf 150°C erhitzt. Nach dem Abkühlen wird die Reaktionsmischung im Vakuum kurz eingeengt und bei 10°C mit 10,2 g Benzoylchlorid tropfenweise versetzt. Nach 30 Minuten Nachreaktion wird die Lösung mit konzentrierter Salzsäure auf pH 2 eingestellt, wobei sich ein weißer Niederschlag bildet. Der Niederschlag wird abfiltriert, mit Wasser nachgewaschen und im Vakuum getrocknet. Umkristallisation aus Benzol liefert 18,7 g N-Benzoylglutaminsäure-$\gamma$-semialdehydpropylen-1,3-acetal (Schmelzpunkt: 157–159°C).

Elementaranalyse: $C_{15}H_{19}NO_5$

| | | | |
|---|---|---|---|
| Berechnet: | C 61,42%, | H 6,53%, | N 4,78%, |
| Gefunden: | C 61,31%, | H 6,41%, | N 4,73%. |

$^1$H-NMR-Spektrum (DMSO-$d_6$/$CDCl_3$)

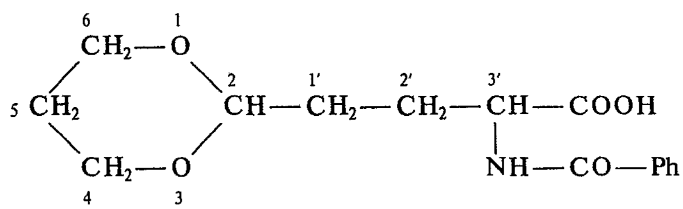

$\delta$ = 1,2–2,2 (m, 6 H): H-1', H-2', H-5
$\delta$ = 3,4–4,2 (m, 4 H): H-4, H-6
$\delta$ = 4,4 (m, 1 H): H-3'
$\delta$ = 7,45; 7,8 (m, 5 H): Aromatische H's
$\delta$ = 8,5 (d, 1 H): N<u>H</u>
$\delta$ = 10,7 (s, 1 H): COO<u>H</u>

## Beispiel 6

Zu einer Suspension aus 20 g Ammoniumcarbonat, 4,8 ml flüssiger Blausäure und 110 ml wäßrigem Ammoniak (25%ig) wird bei 40°C im Verlaufe einer Stunde 17,2 g 2-(2'-Formyläthyl)-5,5-dimethyl-1,3-dioxan, gelöst in 50 ml Methanol, zugetropft, wonach die Temperatur noch 5 Stunden lang auf 40°C gehalten wird. Anschließend werden das Methanol abdestilliert und durch weiteres Erhitzen auf 100°C die Ammoniumsalze verkocht. Die verbleibende wäßrige Suspension wird mit 9 g Natriumhydroxid versetzt und 40 Minuten auf 160°C erhitzt. Nach dem Abkühlen wird die Reaktionsmischung im Vakuum kurz eingeengt und bei 5°C tropfenweise mit 11,2 g Essigsäureanhydrid versetzt, wonach noch 30 Minuten nachgerührt wird. Die Lösung wird mit konzentrierter Salzsäure auf pH 2 eingestellt, wobei sich ein weißer Niederschlag bildet. Der Niederschlag wird abfiltriert, mit Wasser nachgewaschen und im Vakuum getrocknet. Umkristallisation aus Benzol liefert 24,2 g N-Acylglutaminsäure-$\gamma$-semialdehyd-2,2-dimethylpropylen-1,3-acetal (Schmelzpunkt: 168°C–169°C).

Elementaranalyse: $C_{12}H_{21}NO_5$

| | | | |
|---|---|---|---|
| Berechnet: | C 55,58%, | H 8,16%, | N 5,40%, |
| Gefunden: | C 55,91%, | H 8,30%, | N 5,35%. |

$^1$H-NMR-Spektrum (DMSO-$d_6$/$CDCl_3$)

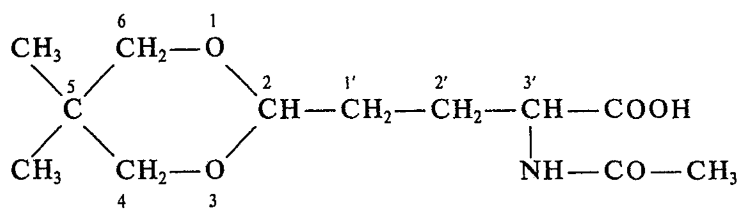

$\delta$ = 0,7 (s, 3 H): 5-$CH_3$
$\delta$ = 1,15 (s, 3 H): 5-$CH_3$
$\delta$ = 1,5–2 (m, 4 H): H-1', H-2'
$\delta$ = 1,92 (s, 3 H): Acetyl
$\delta$ = 3,5 (q, 4 H): H-4, H-6
$\delta$ = 4,2–4,6 (m, 2 H): H-2, H-3'
$\delta$ = 7,7 (d, 1 H): N<u>H</u>
$\delta$ = 11,2 (bs, 1 H): COO<u>H</u>

Beispiel 7

Analog wie im Beispiel 6 werden 17,2 g 2-(2'-Formyläthyl)-5,5-dimethyl-1,3-dioxan umgesetzt. Statt Essigsäureanhydrid werden 14,1 g Benzoylchlorid verwendet. Umkristallisation aus Benzol liefert 31 g N-Benzoylglutaminsäure-$\gamma$-semialdehyd-2,2-dimethylpropylen-1,3-acetal (Schmelzpunkt: 193–194°C).

Elementaranalyse: $C_{17}H_{23}NO_5$

| | | | |
|---|---|---|---|
| Berechnet: | C 63,53%, | H 7,21%, | N 4,36%, |
| Gefunden: | C 63,11%, | H 6,99%, | N 4,28%. |

$^1$H-NMR-Spektrum (DMSO-$d_6$/$CDCl_3$)

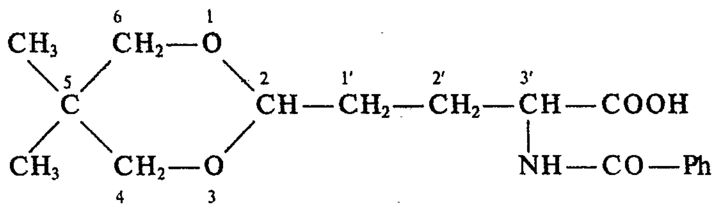

$\delta$ = 0,67 (s, 3 H): 5-$CH_3$
$\delta$ = 1,1 (s, 3 H): 5-$CH_3$
$\delta$ = 1,5–2,1 (m, 4 H): H-1', H-2'
$\delta$ = 3,5 (q, 4 H): H-4, H-6
$\delta$ = 4,2–4,6 (m, 2 H): H-2, H-3'
$\delta$ = 7,4; 7,9 (m, 5 H): Aromatische H's
$\delta$ = 8,5 (d, 1 H): N<u>H</u>
$\delta$ = 12 (bs, 1 H): COO<u>H</u>

Beispiel 8

Analog wie im Beispiel 6 werden 17,2 g 2-(2'-Formyläthyl)-5,5-dimethyl-1,3-dioxan umgesetzt. Statt Essigsäureanhydrid werden 9,8 g Chlorameisensäuremethylester verwendet. Umkristallisation aus Diisopropyläther liefert 22,1 g N-Methoxycarbonylglutaminsäure-$\gamma$-semialdehyd-2,2-dimethylpropylen-1,3-acetal (Schmelzpunkt: 82–83°C).

Elementaranalyse: $C_{12}H_{21}NO_6$

| | | | |
|---|---|---|---|
| Berechnet: | C 52,35%, | H 7,69%, | N 5,09%, |
| Gefunden: | C 52,58%, | H 7,75%, | N 5,04%. |

$^1$H-NMR-Spektrum ($CDCl_3$)

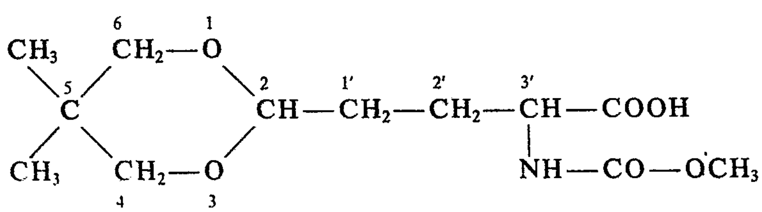

$\delta$ = 0,7 (s, 3 H): 5-$CH_3$
$\delta$ = 1,15 (s, 3 H): 5-$CH_3$
$\delta$ = 1,4–2,1 (m, 4 H): H-1', H-2'
$\delta$ = 3,5 (q, 4 H): H-4, H-6
$\delta$ = 3,65 (s, 3 H): O–$C\underline{H}_3$
$\delta$ = 4,2–4,6 (m, 2 H): H-2, H-3'
$\delta$ = 5,6 (d, 1 H): N$\underline{H}$
$\delta$ = 10,7 (s, 1 H): COO$\underline{H}$

## Beispiel 9

Eine 60°C warme Lösung von 10,85 g N-Acetylglutaminsäure-$\gamma$-semialdehydäthylenacetal, hergestellt nach Beispiel 3, in 100 ml 0,1 N HCl wird innerhalb einer Stunde zu einer 90°C warmen gerührten Lösung von 7,3 g Phenylhydrazinhydrochlorid in 75 ml 0,1 N HCl zugetropft. Das Reaktionsgemisch wird anschließend noch 2,5 Stunden lang auf 90°C gehalten. Nach langsamen Abkühlen auf 10°C wird der entstandene Niederschlag abfiltriert, mit Wasser nachgewaschen und bei vermindertem Druck getrocknet. Die Ausbeute an N-Acetyl-D,L-tryptophan beträgt 8,2 g (66% der Theorie).

## Beispiel 10

Eine Lösung aus 11,55 g N-Acylglutaminsäure-$\gamma$-semialdehydpropylen-1,3-acetal und 7,3 g Phenylhydrazinhydrochlorid in 175 ml 0,1 N HCl wird 3 Stunden lang unter kräftigem Rühren auf 90°C erhitzt. Nach langsamen Abkühlen auf 10°C wird der entstandene Niederschlag abfiltriert und bei vermindertem Druck getrocknet. Die Ausbeute an N-Acetyl-D,L-tryptophan beträgt 7,6 g (62% der Theorie).

## Beispiel 11

Analog wie im Beispiel 10 werden 12,95 g N-Acylglutaminsäure-$\gamma$-semialdehyd-2,2-dimethylpropylen-1,3-acetal und 7,3 g Phenylhydrazinhydrochlorid in N-Acetyl-D,L-tryptophan umgewandelt. Die Ausbeute beträgt 8,5 g (69% der Theorie).

## Beispiel 12

Eine 65°C warme Lösung von 7,5 g N-Benzoylglutaminsäure-$\gamma$-semialdehydäthylenacetal in 100 ml 0,3 N HCl wird innerhalb einer Stunde in eine 90°C warme gerührte Lösung von 4,1 g Phenylhydrazinhydrochlorid in 0,1 N HCl (100 ml) zugetropft und noch 3 Stunden lang bei 90°C gehalten. Es scheidet sich eine ölige Phase ab, die beim Abkühlen zu einer spröden Masse kristallisiert. Umkristallisation aus 80 ml 25%iger wäßriger Essigsäurre liefert 6,2 g N-Benzoyl-D,L-tryptophan. (72% der Theorie).

## Beispiel 13

Analog wie im Beispiel 12 werden 7,3 g N-Benzoylglutaminsäure-$\gamma$-semialdehydpropylen-1,3-acetal aus Beispiel 5 und 3,6 g Phenylhydrazinhydrochlorid in 5,8 g N-Benzoyl-D,L-tryptophan umgewandelt (75% der Theorie).

## Beispiel 14

Analog wie im Beispiel 12 werden 8 g N-Benzoylglutaminsäure-$\gamma$-semialdehyd-2,2-dimethylpropylen-1,3-acetal aus Beispiel 7 und 3,6 g Phenylhydrazinhydrochlorid in 5,4 g N-Benzoyl-D,L-tryptophan umgewandelt (70% der Theorie).

**Patentansprüche**

1. Cyclische Acetale von N-Acylglutaminsäure-γ-semialdehyden der allgemeinen Formel

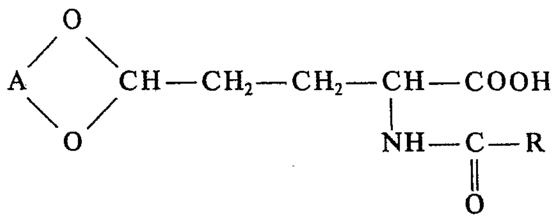

(I)

in der A eine unsubstituierte oder durch 1 bis 2 Methylgruppen substituierte Alkylengruppe mit 2 oder 3 Kohlenwasserstoffatomen und R einen Methyl-, Methoxy-, Phenyl- oder Benzyloxyrest bedeuten.

2. Verfahren zu Herstellung der cyclischen Acetale von N-Acylglutaminsäure-γ-semialdehyden der allgemeinen Formel (I) gemäß Anspruch 1, dadurch gekennzeichnet, daß man

a) eine Verbindung der allgemeinen Formel

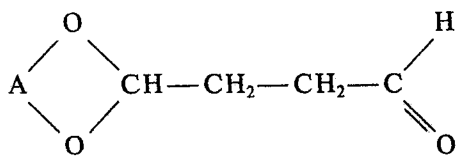

(II)

in der A wieder die bereits angegebene Bedeutung hat, in an sich bekannter Weise in wäßriger oder wäßrig-alkoholischer Lösung mit Cyanwasserstoff oder einer Cyanidionen liefernden Verbindung, Ammoniak oder einer Ammoniumionen liefernden Verbindung und Kohlendioxid oder einer Carbonationen liefernden Verbindung zur Reaktion bringt,

b) das in Stufe a) erhaltene Reaktionsgemisch unter basischen Reaktionsbedingungen hydrolysiert und

c) das in Stufe b) erhaltene Hydrolysegemisch mit einer geeigneten acylierenden Verbindung umsetzt.

3. Verfahren nach Anspruch 2, dadurch gekennzeichnet, daß man in der Stufe a) den Cyanwasserstoff oder die Cyanidionen liefernde Verbindung in einer Menge zwischen 1 und 1,5 Mol, den Ammoniak oder die Ammoniumionen liefernde Verbindung in einer Menge zwischen 2 und 15 Mol und das Kohlendioxid oder die Carbonationen liefernde Verbindung in einer Menge zwischen 1 und 2 Mol, jeweils pro Mol eingesetzter Verbindung der allgemeinen Formel (II), einsetzt.

4. Verfahren nach Anspruch 2 oder 3, dadurch gekennzeichnet, daß man die Stufe a) bei einer Temperatur zwischen 30 und 90°C durchführt.

5. Verfahren nach einem der Ansprüche 2 bis 4, dadurch gekennzeichnet, daß man die Stufe b) bei einer Temperatur zwischen 100 und 150°C durchführt.

6. Verfahren nach einem der Ansprüche 2 bis 5, dadurch gekennzeichnet, daß man die Stufe c) bei einem pH oberhalb von 7 durchführt.

7. Verfahren nach einem der Ansprüche 2 bis 6, dadurch gekennzeichnet, daß man die Stufe c) bei einer Temperatur zwischen 0 und 80°C durchführt.

8. Verwendung der cyclischen Acetale von N-Acylglutaminsäure-γ-semialdehyden der allgemeinen Formel (I) gemäß Anspruch 1 zur Herstellung von N-Acyltryptophanen der allgemeinen Formel

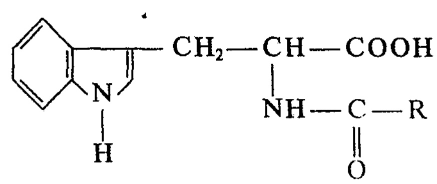

(III)

in der R wieder die bereits angegebene Bedeutung hat.

**Claims**

1. Cyclic acetals of N-acylglutamic acid-γ-semialdehydes corresponding to the general formula

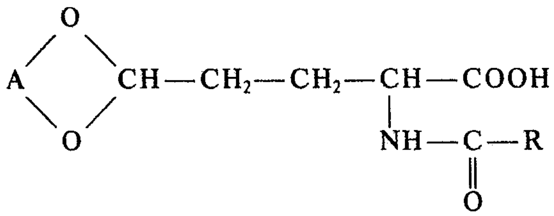

(I)

wherein A represents an alkylene group which is unsubstituted or substituted by from 1 to 2 methyl groups, having 2 or 3 carbon atoms and R represents a methyl, methoxy, phenyl or benzyloxy radical.

2. A process for the production of cyclic acetals of N-acylglutamic acid-γ-semialdehydes corresponding to the general formula (I) according to claim 1, characterised in that

a) a compound corresponding to the general formula

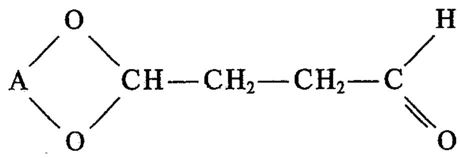

(II)

wherein A is as defined above, is reacted in known manner in aqueous or aqueous-alcoholic solution with hydrogen cyanide or a cyanide ion-producing compound, ammonia or an ammonium ion-producing compound and carbon dioxide or a carbonate ion-producing compound,

b) the reaction mixture which is obtained in stage a) is hydrolysed under basic reaction conditions and

c) the hydrolysis mixture which is obtained in stage b) is reacted with a suitable acylating compound.

3. A process according to claim 2, characterised in that in stage a) the hydrogen cyanide or the cyanide ion-producing compound is used in a quantity of from 1 to 1.5 mols, the ammonia or the ammonium ion-producing compound is used in a quantity of from 2 to 15 mols and the carbon dioxide or the carbonate ion-producing compound is used in a quantity of from 1 to 2 mols, in each case per mol of the compound corresponding to the general formula (II) which is used.

4. A process according to claim 2 or 3, characterised in that stage a) is carried out at a temperature of from 30 to 90°C.

5. A process according to one of claims 2 to 4, characterised in that stage b) is carried out at a temperature of from 100 to 150°C.

6. A process according to one of claims 2 to 5, characterised in that stage c) is carried out at a pH of more than 7.

7. A process according to one of claims 2 to 6, characterised in that stage c) is carried out at a temperature of from 0 to 80°C.

8. The use of the cyclic acetals of N-acylglutamic acid-γ-semialdehydes, corresponding to the general formula (I) according to claim 1 for the production of N-acyltryptophanes corresponding to the general formula

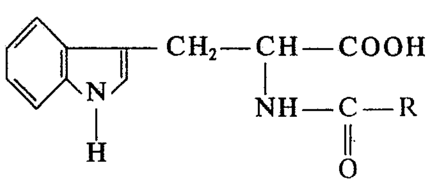

(III)

wherein R is as defined above.

## Revendications

1. Acétals cycliques de semialdéhyde-γ de l'acide N-acylglutamique de formule générale:

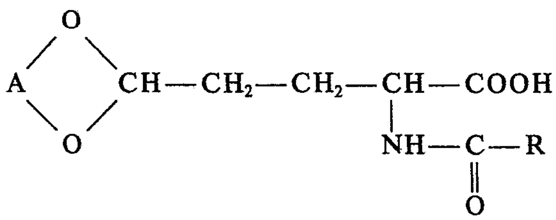

(I)

où A est un groupe alcoylène non substitué ou substitué par 1 à 2 groupes méthyle, comportant 2 à 3 atomes de carbone, et R un reste méthyle, méthoxy, phényl ou benzyloxy.

2. Procédé de préparation des acétals cycliques de semialdéhyde-γ de l'acide N-acylglutamique de formule générale (I), selon la revendication 1, caractérisé en ce que:

a) on fait réagir un composé de formule générale:

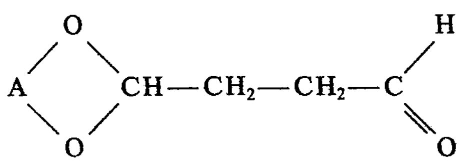

(II)

dans laquelle A a la signification mentionnée plus haut, de manière connue en soi, en solution aqueuse ou hydroalcoolique, avec de l'acide cyanhydrique ou un composé libérant des ions cyanure, de l'ammoniac ou un composé libérant des ions ammonium, et du dioxyde de carbone ou un composé libérant des ions carbonate,

b) on hydrolyse le mélange réactionnel obtenu dans l'étape a), dans des conditions réactionelles basiques, et

c) on fait réagir le mélange d'hydrolyse obtenu dans l'étape b), avec un composé d'acylation approprié.

3. Procédé selon la revendication 2, caractérisé en ce que dans l'étape a), on emploie l'acide cyanhydrique ou le composé libérant des ions cyanure, en une proportion comprise entre 1 et 1,5 mole, l'ammoniac ou le composé libérant des ions ammonium, en une proportion comprise entre 2 et 15 moles, et la dioxyde de carbone ou le composé libérant des ions carbonate, en une proportion comprise entre 1 et 2 moles, toujours par mole de composé de formule générale (II) mis en oeuvre.

4. Procédé selon l'une des revendications 2 ou 3, caractérisé en ce que l'on effectue l'étape a) à une température comprise entre 30 et 90°C.

5. Procédé selon l'une quelconque des revendications 2 à 4, caractérisé en ce que l'étape b) est effectuée à une température comprise entre 100 et 150°C.

6. Procédé selon l'une quelconque des revendications 2 à 5, caractérisé en ce que l'étape c) est effectuée à un pH supérieur à 7.

7. Procédé selon l'une quelconque des revendications 2 à 6, caractérisé en ce que l'étape c) est effectuée à une température comprise entre 0 et 80°C.

8. Utilisation des acétals cycliques de semialdéhyde-γ de l'acide N-acylglutamique, de formule générale (I), selon la revendication 1, pour la préparation des N-acyl-tryptophanes de formule générale:

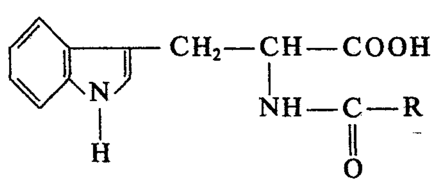

(III)

dans laquelle R a la même signification que plus haut.